## Europäisches Patentamt
## European Patent Office
## Office européen des brevets

(11) Publication number: **0 095 057**
**B1**

(12) **EUROPEAN PATENT SPECIFICATION**

(45) Date of publication of patent specification: **17.11.88**

(51) Int. Cl.⁴: **G 01 N 33/49** // B01D43/00

(21) Application number: **83104215.5**

(22) Date of filing: **11.09.80**

(60) Publication number of the earlier application in accordance with Art. 76 EPC: **0 035 575**

(54) **Blood collecting tube.**

(30) Priority: **11.09.79 JP 116388/79**
**26.05.80 JP 69913/80**

(43) Date of publication of application:
**30.11.83 Bulletin 83/48**

(45) Publication of the grant of the patent:
**17.11.88 Bulletin 88/46**

(84) Designated Contracting States:
**DE NL SE**

(56) References cited:
**FR-A-2 264 594**
**US-A-4 153 739**
**US-A-4 159 896**

(73) Proprietor: **TERUMO KABUSHIKI KAISHA trading as TERUMO CORPORATION**
**44-1, 2-chome, Hatagaya Shibuya-Ku Tokyo 151 (JP)**

(72) Inventor: **Asada, Yoshimitsu**
**25-2, Honmachi 5-chome**
**Shibuya-ku Tokyo 151 (JP)**
Inventor: **Watanabe, Teruko**
**27-13, Himonya 5-chome**
**Meguro-ku Tokyo 152 (JP)**
Inventor: **Ichikawa, Toshiji**
**9-7, Someji 2-chome**
**Chofu-shi Tokyo (JP)**

(74) Representative: **Klöpsch, Gerald, Dr.-Ing.**
**An Gross St. Martin 6**
**D-5000 Köln 1 (DE)**

Courier Press, Leamington Spa, England.

EP 0 095 057 B1

## Description

### Technical field

The present invention relates to a blood collection tube which is capable of promoting coagulation of collected blood.

### Technical background

If the blood is not sufficiently coagulated during centrifugation to separate serum from the collected whole blood, the formation of a blood separating agent layer having an intermediate specific gravity is impaired after the centrifugation. When the blood collection tube is oriented transversely, the blood cells leak into the serum layer or fibrin tends to form. This limits the amount of serum which may be collected.

For this reason, conventionally, the collected blood is sufficiently coagulated over a period of 45 minutes to one hour before centrifugation. Even if this measure is taken, sufficient coagulation may not be achieved due to differences among individuals, and fibrin may precipitate.

Such coagulation over an extended period of time is not preferable from the viewpoints of serum separating and blood testing efficiencies. Therefore, various methods have been proposed to promote coagulation of blood. There are known, for example, a method for coating a glass powder on the inner wall of a blood collection tube utilizing a water-soluble polymer (U.S. Patent No. 4,153,739) or a method for placing a vessel holding a glass powder in the blood collection tube (U.S. Patent No. 4,189,382).

However, with the former method, the steps of coating and drying are complex and cumbersome. Furthermore, since an organic solvent is used, the working environment is not good. With the latter method, since a vessel for holding the glass powder is required, the cost becomes higher. Furthermore, it is difficult to feed a suitable amount of the glass powder.

The present invention has been made in consideration of this and it is an object of the present invention to provide a blood collection tube which is capable of uniformly holding a suitable amount of a blood coagulant, and which presents no problems in the working environment.

In order to achieve this object, there is provided according to the present invention a blood collection tube having a blood coagulation-promoting medium held in said tube with a bottom, characterized in that the blood coagulation-promoting medium comprises a blood coagulation-promoting agent carried on a carrier having a specific gravity which is higher than the specific gravity of blood cells and said blood coagulation-promoting medium being held between the walls of said tube at an intermediate position thereof, and that said carrier has enough rigidity or elasticity to be kept within an intermediate portion of said tube and sufficient flexibility to be movable into a blood cell layer by a centrifugal force and a size which makes it possible to be sunk into a blood cell layer.

Brief explanation of drawings:

Fig. 1 is a perspective view illustrating the blood collection tube having encapsulated the blood coagulation promoting medium.

Fig. 2 is a sectional view of the blood collection tube shown in Fig. 1 after centrifugal separation.

The best modes for carrying out the invention:

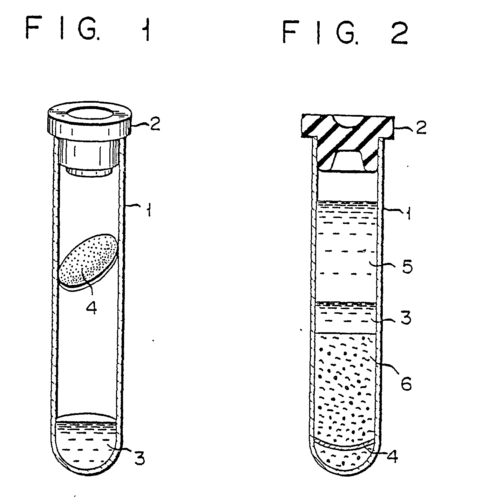

The present invention will now be described by way of examples with reference to the accompanying drawings. Referring to the drawings, Fig. 1 shows a blood collection tube. To the open end of the blood collection tube 1 is fitted a rubber plug 2. At the bottom of the blood collection tube 1 is held a blood (serum) separating agent 3 which consists of a thixotropic gel-like material and which prevents mixing between the serum layer and the blood clot layer of blood.

An apparatus comprising the blood collecting tube and a thixotropic gel-like material is claimed in EP—B—39898.

A blood coagulation-promoting medium 4 is suspended between the walls of the blood collection tube 1 at the intermediate position being kept at this position preferably with the help of the rigidity of the medium. If the blood coagulation-promoting medium 4 is of disc-shape as shown in Fig. 1, it is preferably arranged obliquely at the intermediate position of the blood collection tube 1.

The blood coagulation-promoting medium 4 is obtained by supporting a powderous blood coagulation-promoting agent (preferably by means of a binder which is soluble in the serum) on a carrier which has a specific gravity higher than that of blood cells.

The carrier of the medium 4 may be freely selected from cloth, filter paper, unwoven cloth and cotton wool. However, the carrier must satisfy the following requirements: The carrier must have a specific gravity higher than that of blood cells. The carrier must not cause hemolysis nor affect the biochemical tests. The carrier must be a substance which has the water retention characteristic (e.g., be of hydrophilic nature) or have a water retention structure (e.g., open cell structure). The carrier must have enough rigidity or elasticity to be kept within intermediate portion of a tube, and have a sufficient flexibility to be movable into a blood cell layer by a centrifugal force, and a size which makes it possible to be sunk into a blood cell layer.

There is no limitation on the kind of blood coagulation-promoting agent as far as it does not substantially cause hemolysis and has a buoyancy within blood. For example, it is possible to use powders 0.4 to 20 μm in size of diatomaceous earth, glass, kaolin, and bentonite.

The amount of blood coagulation-promoting agent to be supported on a carrier may be 0.3 to 5.0 mg, preferably 0.3 to 3.5 mg per 10 ml of blood. If the amount of the agent exceeds the upper limit, it may affect the sampled blood due to the dispersion of the powder. On the other hand, if the amount of the agent is less than the lower limit, it would be difficult to expect a sufficient blood coagulation effect.

The amount of the blood coagulation-promoting agent to be supported on a carrier may be suitably adjusted by controlling the mixing ratio of the agent with a binder.

It is required for the binder to be soluble to serum because it is preferable to dissolve and instantly disperse the blood coagulation-promoting agent in blood, as soon as blood is introduced into a blood sampling tube and thereby promoting the formation of clot. It is also required for the binder to have a suitable degree of stickiness and non-volatility, to be hematologically inactive and to be scarcely affected by a sterilizing operation (for example, γ-ray irradiation). Examples of such a binder includes water-soluble silicone, polyethylene glycol, polyvinyl pyrrolidone, dextran, carboxyl methylcellulose, hydroxypropylmethylcellulose, and other cellulose derivatives.

Especially cellulose derivatives and polyvinyl pyrrolidone are superior in solubility and in hygroscopicity and are most preferable, dextran is inferior in solubility as compared with other materials.

Polyethylene glycol has a hygroscopicity and therefore is not suited for storing it in a room temperature for a long period of time after the manufacture thereof.

Methods for manufacturing the medium such as non-woven cloth fixed with a prescribed amount of blood coagulation promoting agent may be easily carried out by a dipping method.

The serum separating agent used may be placed in the blood collection tube as shown in Fig. 1 before or after the blood collection.

Example

As shown in Fig. 1, about 1.7 ml of a liquid separating agent composition 3 was put at the bottom of a 10 ml blood collection tube 1. A polyester unwoven cloth coated with 1—5 mg of diatomaceous earth (e.g., Caper Flattery Sand, trade name WG-200, for Kyoritsu Ceramic Materials Co., Ltd.) or micro-glass powder (blood coagulation-promoting medium 4), was then placed at a slant in each blood collection tube. Each tube was then stopped with a butyl rubber plug 2, and the tubes were placed under reduced pressure. Then a blood sample was placed in each blood collecting tube and allowed to stand for 7 to 8 minutes. As a result, the diatomaceous earth dispersed in the blood upon the introduction of the blood, and it accelerated blood coagulation together with the nonwoven cloth. Adequate coagulation was thus attained within this short time. Each blood collecting tube was placed in a centrifuge for 10 minutes at 700—1,000 G, and the liquid separating agent compositions were stably distributed between the serum and the clot layers. This state is shown in Fig. 2. Because the liquid separating agent is thixotropic and has a specific gravity betwen that of the blood serum 5 and that of the blood clots 6, it stays between the blood serum 5 and blood clot 6, forming a gel that separates these two layers. Since the diatomaceous earth and the unwoven cloth have higher specific gravities, they were not included in the layer of blood serum 5. Thus, blood serum 5 obtained was of high purity with no entrainment of fibrin. This blood serum 5 was readily collected from the blood collecting tube by decantation or by suction through a fine nozzle.

**Claims**

1. A blood collection tube having a blood coagulation-promoting medium held in said tube with a bottom, characterized in that the blood coagulation-promoting medium comprises a blood coagulation-promoting agent carried on a carrier having a water-retention characteristic or a water-retention structure having the specific gravity higher than the specific gravity of blood cells and said blood coagulation promoting medium being held between the walls of said tube at an intermediate position thereof, and that said carrier has enough rigidity or elasticity to be kept within an intermediate portion of said tube and sufficient flexibility to be movable into a blood cell layer by a centrifugal force and a size which makes it possible to be sunk into a blood cell layer.

2. A blood collection tube according to claim 1, characterized in that the blood coagulation-promoting agent is carried in an amount of 0.3 to 5.0 mg per 10 ml of collected blood.

3. A blood collection tube according to claim 1, characterized in that the carrier consists of a hydrophilic substance.

4. A blood collection tube according to claim 1, characterized in that the blood coagulation-promoting agent is carried via a binder which is soluble in serum on the carrier.

5. A blood collection tube according to claim 4, characterized in that the binder is a member selected from water-soluble silicone, polyethylene glycol, dextran, a cellulose derivative, and polyvinyl pyrrolidone.

6. A blood collection tube according to claim 1, characterized in the that the blood coagulation-promoting agent is a member selected from glass, kaolin, bentonite and diatomaceous earth.

**Patentansprüche**

1. Blutsammelröhrchen mit einem blutkoagulationsfördernden Medium, welches in dem Röhrchen mit einem Boden gehalten wird, dadurch gekennzeichnet, daß das blutkoagulationsfördernde Medium ein blutkoagulationsfördernes Mittel enthält, welches auf einem Träger getragen wird mit einer Wasserrückhaltecharakteristik oder einer Wasserrückhaltungsstruktur und einem spezifischen Gewicht, das höher als das spezifische Gewicht von Blutzellen ist, wobei dieses blutkoagulationsfördernde Medium zwischen den Wänden des Röhrchens in einer Zwischenposition gehalten wird, und daß dieser Träger genügend Festigkeit oder Elastizität aufweist, um in dem Mittelteil gehalten zu werden und genügend Flexibilität, um durch Zentrifugalkraft in eine Blutzellenschicht bewegt werden zu können, und eine Größe, die es ermöglicht, in die Blutzellenschicht einzusinken.

2. Blutsammelröhrchen nach Anspruch 1, dadurch gekennzeichnet, daß das blutkoagulationsfördernde Mittel in einer Menge von 0,3 bis 5,0 mg pro 10 ml gesammelten Bluts getragen wird.

3. Blutsammelröhrchen nach Anspruch 1, dadurch gekennzeichnet, daß der Träger aus einer hydrophilen Substanz besteht.

4. Blutsammelröhrchen nach Anspruch 1, dadurch gekennzeichnet, daß das blutkoagulationsfördernde Mittel über ein Bindemittel, das im Serum löslich ist, auf dem Träger getragen wird.

5. Blutsammelröhrchen nach Anspruch 4, dadurch gekennzeichnet, daß das Bindemittel ein aus wasserlöslichem Silikon, Polyethylenglykol, Dextran, einem Cellulosederivat und Polyvinylpyrrolidon ausgewähltes Element ist.

6. Blutsammelrörchen nach Anspruch 1, dadurch gekennzeichnet, daß das blutkoagulationsfördernde Mittel ein aus Glas, Kaolin, Bentonit und Kieselgur ausgewähltes Element ist.

## Revendications

1. Tube pour recueillir le sang, fermé à une extrémité (fond) et contenant un produit favorisant la coagulation du sang, caractérisé en ce que le produit favorisant la coagulation du sang est constitué d'un agent favorisant la coagulation sanguine déposé sur un support possédant des propriétés de ou une structure lui permettant la rétention d'eau et dont la masse volumique spécifique est supérieure à celle des globules sanguins, ledit produit favorisant la coagulation du sang étant placé et maintenu dans ledit tube en une position intermédiaire (entre les deux extrémités) et ledit support possédant une rigidité et(ou) élasticité adéquate pour être retenu dans cette position et une flexibilité suffisante pour être amené par une force centrifuge dans la couche de globules sanguins, ses dimensions étant telles qu'il puisse être amené dans cette couche de globules sanguins.

2. Tube pour recueillir le sang suivant la revendiation 1, caractérisé en ce que la proportion de l'agent favorisant la coagulation sanguine, porté par le support, est de l'ordre de 0,3 à 5,0 mg pour 10 ml de sang recueilli.

3. Tube pour recueillir le sang suivant la revendication 1, caractérisé en ce que le support est une substance hydrophile.

4. Tube pour recueillir le sang suivant la revendication 1, caractérisé en ce que l'agent favorisant la coagulation du sang est fixé au support à l'aide d'une liant soluble dans le sérum sanguin.

5. Tube pour recueillir le sang suivant la revendication 4, caractérisé en ce que le liant est choisi parmi les substances hydrosolubles telles qu'une silicone, le polyéthylène-glycol, le dextrane, un dérivé cellulosique et la polyvinyl-pyrrolidone.

6. Tube pour recueillir le sang suivant la revendication 1, caractérisé en ce que l'agent favorisant la coagulation sanguine est choisi parmi le verre, les kaolins, les bentonites et les terres de diatomées.

EP 0 095 057 B1

F I G. 1

F I G. 2

1